# EUROPEAN PATENT APPLICATION

(11) **EP 2 556 844 A1**
(43) Date of publication of application: **13.02.2013**
(21) Application number: 11765328.7
(22) Date of filing: 15.03.2011
(51) Int. Cl.: A61L 27/00

(54) **METHOD FOR RESTORING ALVEOLAR BONE VIA TRANSPLANT OF A REGENERATED TOOTH UNIT**

(30) Priority: 01.09.2010 JP 2010196016; 07.04.2010 JP 2010088967
(71) Applicant: Organ Technologies Inc., Chiyoda-ku Tokyo 101-0048 (JP)
(72) Inventor: TSUJI, Takashi, Tokyo 162-8601 (JP); NAKAO, Kazuhisa, Tokyo 162-8601 (JP); OSHIMA, Masamitsu, Tokyo 162-8601 (JP)
(74) Representative: Stephen, Robert John
(86) International application number: PCT/JP2011/056008
(87) International publication number: WO 2011/125426

(57) **Abstract**

Problem to be Solved

The object of the present invention is to provide a method for restoring the alveolar bone of a mammal with a missing tooth.

Solution

The present invention provides a method for restoring the alveolar bone in a mammal with a missing tooth comprising a step of transplanting a regenerated tooth unit to the said missing site. The regenerated tooth unit is characterized in that it has a periodontal tissue portion in addition to a tooth crown portion.

## Description

### Technical Field

The present invention relates to a method for restoring the alveolar bone via transplantation of a regenerated tooth unit, etc.

### Background Art

The tooth is supported by the periodontal tissue present around the tooth. The tooth itself is an organ that has hard tissues of enamel on the outermost layer and dentin on the inner layer thereof, odontoblast which produces dentin further inside, and dental pulp at the center. On the other hand, the periodontal tissue has a bone called the alveolar bone as the base, the cementum and the periodontal ligament exist around the tooth root where the alveolar bone is in contact with the tooth root, and the gingiva is formed so that it covers the outer layer of the alveolar bone other than the portion in contact with the tooth root. Diseases that occur in these periodontal tissues are for example gingivitis where inflammation occurs in the gingiva, periodontitis where inflammation occurs further in the periodontal tissue, and alveolar pyorrhea where further the alveolar bone is lysed and decreased. The lysis of the alveolar bone will make it difficult to support the tooth, and result in loss of tooth. Supporting the tooth will also become difficult when alveolar bone resorption is progressed due to aging.
On the other hand, a tooth is often lost and implant therapy is performed in the case of a disease of the tooth per se. In this case, the alveolar bone that supports the implant is necessary in order to transplant the implant, but implant therapy cannot be performed if the alveolar bone is not sufficient. It is also known that even if there is sufficient bone tissue at the time of implant therapy, resorption of the alveolar bone will progress after transplantation of the implant.
Accordingly, there is an increased interest towards technology for restoring or regenerating lost alveolar bone.

In recent years, GTR method (guided tissue regeneration) or Emdogain® etc. are sometimes employed as periodontal disease therapies. The GTR method is a method that attempts to prevent the invasion of the gingiva epithelium into the alveolar bone side and promote the vertical regeneration of periodontal tissues such as the periodontal ligament and the alveolar bone by inserting a membrane after periodontal surgery. In addition, Emdogain® is an agent that has enamel matrix protein as the main component, and is a method to attempt regeneration of destroyed periodontal tissue. However, it is difficult to promote vertical regeneration of the alveolar bone with either method, and its effect is not sufficient especially for regeneration of a large alveolar bone.

The present inventors focused on tooth regeneration, and have thus far found that by employing mesenchymal cells and epithelial cells wherein at least either one of these cells is tooth germ-derived cells, and disposing a first cell aggregate consisting substantially of mesenchymal cells and a second cell aggregate consisting substantially of epithelial cells in contact inside a support carrier consisting of e.g. collagen gel, and then culturing the first and second cell aggregates inside the support carrier, a regenerated tooth germ or regenerated tooth unit having effectively induced cell differentiation as well as specific cell disposition and direction can be produced (see e.g. Patent Document 1).

Further, it was also shown that a regenerated tooth germ or regenerated tooth unit having specific cell disposition and direction is similarly obtained when oral epithelial cells or primary cultured cells thereof are utilized as the epithelial cells (see e.g. Patent Document 2), when amnion-derived cells are utilized as the mesenchymal cells (see e.g. Patent Document 3), and when cells differentiation-induced from totipotent stem cells are utilized as the mesenchymal cells (see e.g. Patent Document 4). It was also shown that these regenerated teeth can be utilized similarly to innate teeth in regards to occlusion and hardness (see e.g. Patent Document 5).

Patent Document 1
   International Publication No. 2006/129672
Patent Document 2
   Japanese Published Unexamined Patent Application Publication No. 2008-29756
Patent Document 3
   Japanese Published Unexamined Patent Application Publication No. 2008-206500
Patent Document 4
   Japanese Published Unexamined Patent Application Publication No. 2008-200033
Patent Document 5
   International Publication No. 2010/021340

### Summary of the Invention

### Problems to be Solved by the Invention

The object of the present invention is to restore the alveolar bone of a mammal with a missing tooth.

### Means for Solving the Problems

As a result of repeated research to solve the above problems, the present inventors found that the alveolar bone can be restored compared to before transplantation of a regenerated tooth unit by transplanting a regenerated tooth unit to the missing tooth site in a mammal with a missing tooth.
In other words, the present invention relates to a method for restoring the alveolar bone of a mammal with a missing tooth, comprising a step of transplanting a regenerated tooth unit to said missing tooth site.
One embodiment of the method for restoring the alveolar bone of a mammal with a missing tooth of the present invention is characterized in that said animal is a non-human mammal.
One embodiment of the method for restoring the alveolar bone of a mammal with a missing tooth of the present invention is characterized in that it comprises the steps of:
(a) producing a regenerated tooth germ;
(b) culturing said regenerated tooth germ in vivo in the body of a mammal, and producing a regenerated tooth unit; and
(c) transplanting said regenerated tooth unit to the missing tooth site of the mammal.
   One embodiment of the method for restoring the alveolar bone of a mammal with a missing tooth of the present invention is characterized in that it comprises the steps of:
   (a) producing a regenerated tooth germ;
   (b) culturing said regenerated tooth germ in vivo in the body of a mammal which is an individual that is different from the subject for jaw bone restoration, and producing a regenerated tooth unit; and
   (c) transplanting said regenerated tooth unit to the missing tooth site of a mammal that will be the subject for jaw bone restoration.
One embodiment of the method for restoring the alveolar bone of a mammal with a missing tooth of the present invention is characterized in that said regenerated tooth germ is cultured and maintained by disposing a first cell assembly composed substantially of mesenchymal cells and a second cell assembly composed substantially of epithelial cells in close contact inside a support carrier, wherein at least either one of mesenchymal and epithelial cells is tooth germ-derived.
In another embodiment of the method for restoring the alveolar bone of a mammal with a missing tooth of the present invention is characterized in that
said (b) step is culturing the regenerated tooth germ inside a spacer disposed in vivo in the body of a mammal.
Another embodiment of the method for restoring the alveolar bone of a mammal with a missing tooth of the present invention is characterized in that
mechanical stimulation is applied from the exterior of the mammal in said (b) step.
According to another aspect of the present invention, the present invention also relates to a regenerated tooth unit for restoring the alveolar bone for employing in said method for restoring the alveolar bone.
According to another aspect of the present invention, the present invention also relates to a regenerated tooth germ for producing the alveolar bone for employing in said method for restoring the alveolar bone.

### Advantages of the Invention

According to the present invention, the alveolar bone of a mammal can be restored by transplanting a regenerated tooth unit. In particular, the present invention, as one aspect thereof, enables restoration of the alveolar bone in the vertical direction that was difficult with conventional technology.
In addition, as one aspect of the present invention, according to a method of transplanting a regenerated tooth unit produced with a spacer, it is possible to obtain a regenerated tooth unit in which the periodontal tissue is widely developed around the regenerated tooth without excessive pressure being applied from a tissue etc. of the said animal even when the regenerated tooth germ is cultured in vivo in the body of an animal, and the alveolar bone of the transplanted animal can be better restored by transplanting the said regenerated tooth unit. In addition, as one aspect of the present invention, according to a method of transplanting a regenerated tooth unit produced with a spacer, a long-term culturing is possible so as to promote the development of the periodontal tissue while preventing the tooth from extending to more than necessary, and the alveolar bone of the transplanted animal can be better restored by transplanting the said regenerated tooth unit. In addition, as one aspect of the present invention, by adjusting the size or shape of the spacer according to the size or shape of the missing tooth part and transplanting a regenerated tooth unit cultured and produced with the said spacer, transplantation of a regenerated tooth unit is more easily succeeded, and the alveolar bone of the animal to which the said regenerated tooth unit is transplanted can be better restored.
Further, as one aspect of the present invention, according to a method of transplanting a regenerated tooth unit produced with application of mechanical stimulation, a regenerated tooth having sufficient periodontal ligament tissue can be obtained even when the regenerated tooth germ is cultured in vivo in the body of an animal, and the alveolar bone of the transplanted animal can be better restored by transplanting the said regenerated tooth unit.

### Brief Description of the Drawings

Figure 1 is, as one aspect of the present invention, the photograph showing the appearance of performing in vivo culturing of regenerated tooth germ inside a spacer disposed in the mouse subrenal capsule when manufacturing the regenerated tooth unit with a spacer;
Figure 2 is the micro CT image of a regenerated tooth unit produced with a spacer;
Figure 3 is the micro CT image of a regenerated tooth unit produced without a spacer;
Figure 4 shows the result of the major/minor axis ratios of a regenerated tooth unit produced with a spacer (controlled), a regenerated tooth unit produced without a spacer (uncontrolled), and the tooth crown portion of a natural tooth as measured and calculated on CT images;
Figure 5 shows the result of the lengths of a regenerated tooth unit produced with a spacer (controlled) and a regenerated tooth unit produced without a spacer (uncontrolled) as measured on CT images;
Figure 6 is the HE staining image of a regenerated tooth unit produced with application of mechanical stimulation during in vivo culturing of regenerated tooth germ and a regenerated tooth unit produced without applying stimulation;
Figure 7 is the result of the widths of the periodontal ligament of a regenerated tooth unit produced with application of mechanical stimulation during in vivo culturing of regenerated tooth germ and a regenerated tooth unit produced without applying stimulation as measured on CT images;
Figure 8 shows the appearance of jaw bone transplantation of a regenerated tooth unit manufactured with the method of the present invention;
Figure 9 is the CT image on Day 0, Day 14, and Day 30 after jaw bone transplantation of a regenerated tooth unit manufactured with the method of the present invention;
Figure 10 is the HE staining image on Day 30 after jaw bone transplantation of a regenerated tooth unit manufactured with the method of the present invention;
Figure 11 is the plane view and side view schematically showing a regenerated tooth germ in the state of being disposed inside a spacer;
Figure 12 is the (a) stereoscopic image, (b) CT image, and (c) CT cross-sectional image of the regenerated tooth unit to be transplanted to the large missing bone model;
Figure 13 is the photograph of around the missing site (transplantation site) after 0, 14, 30, and 40 days has passed after transplantation in that order from the left, where the top row is when a regenerated tooth unit is not transplanted to the large missing bone model, and the bottom row is when a regenerated tooth unit is transplanted to the large missing bone model, respectively;
Figure 14 is the photograph of around the missing site (transplantation site) on 45 days after transplantation, where the left is the control, i.e. when a regenerated tooth unit is not transplanted to the large missing bone model, and the right is the transplantation example, i.e. when a regenerated tooth unit is transplanted to the large missing bone model. In the photographs, the dotted line shows the top edge line of the alveolar bone at the time of transplantation, and the solid line shows the top edge line of the alveolar bone 45 days after transplantation;
Figure 15 is the graph showing the regenerated alveolar bone mass for 45 days after transplantation. The bone mass of regenerated alveolar bone is shown, where the left side of the graph is the control, i.e. when a regenerated tooth unit is not transplanted to the large missing bone model, and the right side of the graph is the transplantation example, i.e. when a regenerated tooth unit is transplanted to the large missing bone model; and

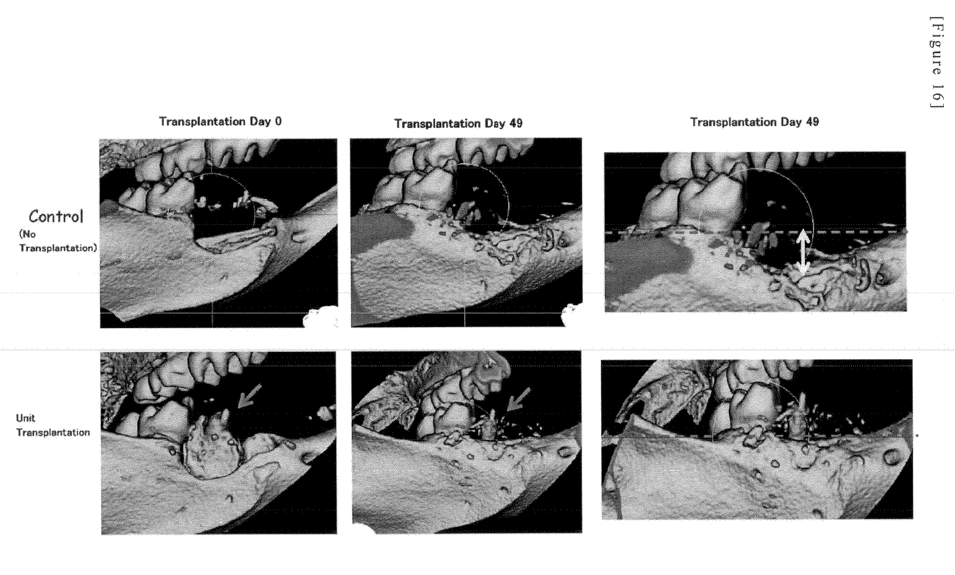

Figure 16 is the CT appearance image of around the missing site (transplantation site), where the top row is the control, i.e. when a regenerated tooth unit is not transplanted to the large missing bone model, and the bottom row is the transplantation example, i.e. when a regenerated tooth unit is transplanted to the large missing bone model. For each of these, the image on the left shows 0 days after transplantation, and the image in the middle shows 49 days after transplantation. The image on the right is the magnified view of the CT image 49 days after transplantation, and for both top and bottom rows, the horizontal line through the top edge of the alveolar bone of the missing site (transplantation site) at 49 days after transplantation in the transplantation example is shown with a dotted line. In the image in the right top row, the difference in the vertical direction between the top edge of the alveolar bone of the control missing site and the top edge of the alveolar bone of the transplantation example is schematically shown with a two-headed arrow.

### Description of Embodiments

The first aspect of the method for restoring the jaw bone according to the present invention is a method for restoring the alveolar bone of an animal missing a tooth, characterized in that it comprises a step of transplanting a regenerated tooth unit to said missing tooth site.

A "tooth" herein refers to a tissue comprising continuous layers of dentin on the inside and enamel on the outside, and means a tissue having a tooth crown or tooth root provided with direction. The direction of a tooth can be specified by the positioning of the tooth crown or tooth root. The tooth crown or tooth root can be visually identified based on for example the shape or tissue staining. The tooth crown refers to the portion having the layered structure of enamel and dentin, and no enamel layer exists in the tooth root.

Dentin and enamel can be easily morphologically specified by those skilled in the art by e.g. tissue staining. Enamel can also be specified by the presence of ameloblast, and the presence of ameloblast can be verified by the presence or absence of amelogenin. On the other hand, dentin can be specified by the presence of odontoblast, and the presence of odontoblast can be verified by the presence or absence of dentin sialoprotein. The verification of amelogenin and dentin sialoprotein can be easily carried out by methods known in the art, examples of which include in situ hybridization and antibody staining.

The "missing" of a tooth herein refers to a state that a tooth is not present in a place that it should be, regardless of the reason for missing. This includes when a tooth drops out naturally due to a disease such as periodontal disease, as well as when artificial tooth extraction is performed for dental therapy etc. "Missing tooth site," "missing site," or "missing part" mean a portion established in the gingiva by a missing tooth such as by tooth extraction, and there is no particular restriction to the shape. In addition, a "missing tooth site" also includes for example a hole created by the missing of a tooth per se, as well as when the shape of the missing site is artificially changed by acts such as grinding of the alveolar bone for implant therapy etc. or when the shape etc. of the missing site is naturally changed by for example resorption of the alveolar bone. In addition, the missing location and the intended type of tooth are not particularly limited as long as the regenerated tooth unit can be embedded. Moreover, this may be referred to "large missing" or "diffuse missing" herein when a large tooth such as the molar is missing, when consecutive multiple small teeth are missing, or when a range corresponding to these is missing regardless of the number of teeth. The missing part is ordinarily located in the jaw bone or the oral alveolar bone.

In the method for restoring the alveolar bone according to the present invention, the transplantation subject of a regenerated tooth unit, i.e. the mammal with a missing tooth is not particularly limited as long as it is a mammal that intrinsically has teeth. It is preferred that the animal missing a tooth is the same species as the animal from which the tooth germ employed for manufacturing the regenerated tooth was harvested, further preferably the same individual as the individual from which the tooth germ was harvested. Examples include a cow, a horse, a pig, a dog, a cat, and a mouse. It is also preferably a non-human mammal.

A "regenerated tooth unit" herein refers to a unit consisting of a tooth and the periodontal tissue supporting the tooth, which is obtained as a result of culturing a regenerated tooth germ and differentiating various cells comprised in the regenerated tooth germ.
The configuration of the tooth portion in the regenerated tooth unit is in the stage between a tooth bud that is in the developmental stage of the tooth described below and a tooth. In addition, the periodontal tissue comprises the periodontal ligament and the alveolar bone. The method for manufacturing the "regenerated tooth unit" is not particularly limited as long as the unit comprises the regenerated tooth portion and the regenerated periodontal tissue portion. As one method for producing a regenerated tooth unit, a regenerated tooth unit can be obtained by culturing a regenerated tooth germ in vivo in the body of a mammal. As another method for producing a regenerated tooth unit, a regenerated tooth unit can be obtained by organ-culturing a regenerated tooth germ for somewhat a long period of time.

The alveolar bone can be easily morphologically specified by those skilled in the art by e.g. visual observation or CT image. CT image or CT cross-sectional image can also be easily taken by those skilled in the art by employing a well-known apparatus. For example, a 3D micro X-ray CT R mCT for experimental animals (Rigaku Corporation) can be employed for taking a CT image, and this can be performed for example under conditions such as 90 kV, 150 mA, and section thickness of 10 mm. Those skilled in the art can also perform e.g. image construction and analysis with an appropriate image analysis software after taking the CT image. Examples of an image analysis software that can be employed include image filing software for small animals i-VIEW Type R and high definition 3D/4D image analysis software Imaris (Bitplane). In addition to the above exemplifications, those skilled in the art can also set the appropriate conditions employing similar instruments, and take CT images and analyze utilizing a similar image analysis software.

The periodontal ligament and the cementum can also be easily morphologically specified by e.g. tissue staining. For example, ordinary hematoxylin-eosin (HE) staining can be employed in the staining method. In performing tissue staining, those skilled in the art can perform HE staining by going through the steps of for example fixing the sample in 4 % paraformaldehyde (Paraformaldehyde: PFA), decalcifying in 10 % ethylenediaminetetraacetic acid (EDTA), paraffin embedding, and then creating continuous sections of 8 micrometers. In addition to the above exemplifications, those skilled in the art can also perform tissue staining and histological evaluation according to a general method.

A "tooth germ" herein is an early germ of a tooth that is defined to be a tooth in the future, and refers to those in the stages from the bud stage to the bell stage generally employed in the developmental stage of the tooth, in particular a tissue in which accumulation of dentin and enamel which are the characteristics as the hard tissues of the tooth is not observed. On the other hand, a "tooth bud" herein refers to a tissue at a later stage then the "tooth germ," and refers to a tissue after the stage where accumulation of dentin and enamel which are the characteristics of the hard tissues of the tooth has begun and before the stage where the tooth erupts from the gingiva and generally expresses the functions as a tooth.
A "regenerated tooth germ" herein refers to a tooth germ artificially formed by a cell culture technology.

"Transplantation" herein refers to disposing and fixing a regenerated tooth unit to a missing tooth site. Upon transplantation, it is preferred to position the periodontal tissue site of the regenerated tooth unit in the missing part, and to position the tooth crown portion of the tooth towards the interior of the mouth. After disposing the regenerated tooth unit at the missing part, it is preferred to carry out suturing etc. according to an ordinary treatment. Moreover, if the loss of tooth is accompanied by reduction in the alveolar bone mass, a well-known method clinically employed for embedding an implant such as the GTR method (guided tissue regeneration: tissue regeneration induction method) may be used to the missing site in combination.

The "restoration" of the alveolar bone herein means the increase in the bone mass of the missing site compared to before transplantation of a regenerated tooth unit. The change in bone mass within a certain range can be measured for example by a method of taking micro CT images of the site to be measured the change in bone mass over time, and performing analysis by an integrated image processing software in order to measure the volume of the bone at the said site before and after transplantation. For example, the change in bone mass of the alveolar bone of the present invention can be measured with such a method by measuring the volume of the alveolar bone in the buccal alveolar bone of the missing site before and after transplantation. It is also possible to morphologically confirm the restoration of the alveolar bone. In other words, for the lower jaw, the top edge portion of the alveolar bone grows towards the upper jaw direction and is in a protruded state compared to before transplantation, and oppositely, for the upper jaw, the bottom edge portion of the alveolar bone grows towards the lower jaw direction and is in a protruded state compared to before transplantation. The restoration of the alveolar bone can also be referred to as the regeneration of the alveolar bone. Such restoration or regeneration of the alveolar bone may be referred to herein as restoration or regeneration of the alveolar bone in the vertical direction, or vertical restoration or regeneration of the alveolar bone. In addition, since the alveolar bone is a term that refers to the site around a tooth in the jaw bone and the extent of the alveolar bone cannot be strictly discriminated, the restoration of the alveolar bone herein may include the restoration of the jaw bone around the alveolar bone.

The regenerated tooth unit employed in the method of the present invention will now be described.
A regenerated tooth unit can form the periodontal tissue around a regenerated tooth by producing and culturing a regenerated tooth germ. The regenerated tooth germ may be those produced by any method, for example, it can be produced by a method comprising the steps of: employing mesenchymal cells and epithelial cells wherein at least either one of these cells is tooth germ-derived cells, and disposing a first cell assembly composed substantially of mesenchymal cells and a second cell assembly composed substantially of epithelial cells in close contact; and culturing the first and second cell assemblies inside a support carrier. In the present specification, although the cell assembly composed substantially of mesenchymal cells is referred to as the first cell assembly for convenience in order to discriminate the two cell assemblies, it is also possible to refer to the cell assembly composed substantially of epithelial cells as the first cell assembly, and it is not essential which of these is referred to as the first cell assembly.

A "mesenchymal cell" in the present invention means a mesenchymal tissue-derived cell and a cell obtained by culturing this cell, and an "epithelial cell" means an epithelial tissue-derived cell and a cell obtained by culturing this cell.
In addition, a "periodontal tissue" in the present invention refers to the alveolar bone and the periodontal ligament formed mainly on the outer layer of the tooth. The alveolar bone and the periodontal ligament can be easily morphologically specified by those skilled in the art by e.g. tissue staining.

The "step of disposing a first cell assembly composed substantially of mesenchymal cells and a second cell assembly composed substantially of epithelial cells in close contact" is for example described in Patent Documents 1 to 4 and Japanese Published Unexamined Patent Application Publication No. 2008-29757, the disclosures of each of these references incorporated herein by reference in its entirety.

The above first cell assembly and second cell assembly are each composed substantially of mesenchymal cells only or epithelial cells only. "Composed substantially of mesenchymal cells only" in the present invention means that one cell assembly is in a state that serves the same function as when it is composed of mesenchymal cells only, and comprises as little as possible anything other than cells that become a mesenchymal cell. The same goes in the case of "consisting substantially of epithelial cells only."
A cell assembly here refers to the state of cells in close contact, and may be a tissue or a cell aggregate prepared from loose cells. Employing a tissue has the advantage that a tooth with correct cell disposition or shape will be easily obtained, but the available amount may be limited. Cell aggregates are relatively easy to obtain and preferred because cultured cells can also be employed. According to the method of the present invention, a regenerated tooth with correct cell disposition or shape can be obtained even when a cell aggregate is employed.

At least either one of mesenchymal and epithelial cells configuring a cell assembly is tooth germ-derived. This allows the cell disposition in vivo to be reproduced, and a tooth having specific structure and direction can be effectively formed. It is more preferred that both mesenchymal and epithelial cells are tooth germ-derived. The said tooth germ is preferably one from the bud stage to cap stage with respect to the blastogenicity and homogenicity of cell differentiation stage.

As mesenchymal cells derived from other than the tooth germ, cells derived from other mesenchymal tissues in the body can also be employed. These are preferably blood cell-free bone marrow cells or mesenchymal cells, further preferably oral mesenchymal cells or bone marrow cells inside the jaw bone, mesenchymal cells derived from cephalic neural crest cells, and mesenchymal progenitor cells that may differentiate into these mesenchymal cells or stem cells thereof etc. An example of employing amnion-derived cells as the mesenchymal cells is described in Patent Document 3, and an example of employing cells differentiation-induced from totipotent stem cells is described in Patent Document 4, the disclosures of which are incorporated herein by reference in its entirety.

As epithelial cells derived from other than the tooth germ, cells derived from other epithelial tissues in the body can also be employed. These are preferably skin or oral mucosa or gingival epithelial cells, further preferably immature epithelial progenitor cells that may differentiate into differentiated, e.g. keratinized or parakeratinized epithelial cells such as skin or mucosa, for example non-keratinized epithelial cells or a stem cells thereof etc. An example of employing oral epithelial cells or primary cultured cells thereof as the epithelial cells is described in Patent Document 2, the disclosure of which is incorporated herein by reference in its entirety.

The tooth germ and other tissues can be collected from the jaw bone etc. of various animals such as mammalian primates (such as humans and monkeys), ungulates (such as pigs, cows, and horses), small mammalian rodents (such as mice, rats, and rabbits), as well as dogs and cats. Conditions ordinarily employed for tissue collection may be applied without change for collecting the tooth germ and tissue, and may be removed in sterile condition and preserved in an appropriate preservation solution. Examples of a human tooth germ include the tooth germ of the third molar, the so-called wisdom tooth, as well as the fetal tooth germ, but it is preferred to employ the tooth germ of the wisdom tooth with respect to utilization of autologous tissue. In case of a mouse, it is preferred to employ the tooth germ of Embryonic Day 10 to 16.

The preparation of mesenchymal and epithelial cells from a tooth germ is performed by first separating the tooth germ isolated from the surrounding tissue into the tooth germ mesenchymal tissue and the tooth germ epithelial tissue according to its shape. In doing so, an enzyme may be employed to facilitate separation. Examples of an enzyme include dispase, collagenase, and trypsin.

The cell aggregate according to the present invention means aggregated cells derived from the mesenchymal tissue or the epithelial tissue, and can be prepared by aggregating cells obtained by dispersing the mesenchymal tissue or epithelial tissue into bits, or cells obtained by primary or passage culturing of the said cells.

An enzyme such as dispase, collagenase, and trypsin may be employed to disperse cells. When performing primary or passage culturing of dispersed cells before preparing the cell aggregate in order to obtain a sufficient number of cells, the medium employed for culturing that can be employed is a medium generally employed for animal cell culturing such as Dulbecco's Modified Eagle medium (DMEM). A serum for promoting cell proliferation may be added, or a serum alternative, for example a cell growth factor such as FGF, EGF, and PDGF or a known serum component such as transferrin may be added. The concentration when adding a serum can be arbitrarily altered depending the culture state at the time, but is ordinarily around 10 %. For cell culturing, ordinary culture conditions such as culturing in an incubator at a temperature of about 37°C and 5 % CO₂ concentration are applied. An antibiotic such as streptomycin may also be added as appropriate.

In order to aggregate cells, for example, the cell suspension may be centrifugated. It is preferred that the cell aggregates of each of mesenchymal and epithelial cells are kept at a high density state in order to ensure cellular interaction when the two are in close contact. High density state refers to an extent equivalent to the density of constituting a tissue, for example, 5 x 10⁷ cells/ml to 1 x 10⁹ cells/ml, preferably 1 x 10⁸ cells/ml to 1 x 10⁹ cells/ml, and most preferably 2 x 10⁸ cells/ml to 8 x 10⁸ cells/ml. The method for making the cell aggregate high density is not particularly limited, for example it can be performed by a method of aggregating and precipitating the cells by centrifugation. Centrifugation is preferred because it easily enables high density without impairing cellular activity. Centrifugation may be performed at a rotational speed that renders a centrifugal force of 300 x g to 1200 x g, preferably 500 x g to 1000 x g for 3 to 10 minutes. There is a tendency that cell density is unable to be sufficiently increased at centrifugation lower than 300 x g. On the other hand, cells may be damaged at centrifugation higher than 1200 x g.

Ordinarily, when preparing a high density cell aggregate by centrifugal separation, a cellular suspension is prepared in a container such as a tube employed for centrifugal separation of cells before performing centrifugal separation, and the supernatant may be removed as much as possible, leaving the cells as the precipitate. Here, components other than the cells of interest (e.g. culture medium and buffer) are preferably at an amount less than or equal to the cell volume, and most preferably no component other than the cells of interest is comprised. If such high density cell assembly is put into close contact inside a support carrier by the method described below, cells come into tight contact and intercellular interaction is effectively exerted.

The support carrier employed for the purpose of culturing the first and second cell assemblies is preferably equipped with retentivity that enables retaining the cell assemblies in close contact without the cells being dispersed. In close contact means the state where the high density cell assemblies of mesenchymal and epithelial cells described above also retains a similar extent of density near the contact surface of the mesenchymal and the epithelial cells. The support carrier that enables retention of close contact, in case of collagen for example, is used at a concentration of 2 mg/ml to 3 mg/ml final concentration, i.e. a concentration that gives 120 g to 250 g jelly strength by the method in compliance to JIS-K6503-1996 (measured as the load necessary to push down 4 mm with a 12.7 mm diameter plunger) to provide an appropriate hardness. Other types of support carriers are also preferably employed as the support carrier of the present invention if it has a similar strength by a similar evaluation method. A support carrier having a hardness corresponding to the target jelly strength may also be obtained by mixing one or more types of support carriers. A support carrier identical to that disposed inside the spacer described below can be used as the support carrier.

The method of disposing the first and second cell assemblies into the support carrier is not particularly limited, and when the cell assembly is a cell aggregate, for example, the precipitate obtained with the centrifugal separation described above can be inserted into the support carrier with a microsyringe etc. and disposed. When the cell assembly is a tissue, it can be disposed at any position inside the support carrier by employing the tip of a needle of a syringe etc.

The method for disposing the first and second cell assemblies in close contact with the support carrier in the present invention is not particularly limited, for example, the two can be put into close contact by disposing one cell assembly inside the support carrier, and then disposing the other cell assembly so as to push onto it. More specifically, by arbitrarily altering the position of the above tip of the needle of the syringe inside the support carrier, one cell assembly can be pushed onto the other cell assembly. When employing an epithelial or a mesenchymal tissue as the cell assembly, it is preferred to dispose the surface of the said tissue that had contacted the mesenchymal or the epithelial tissue in the original tooth germ in contact with the other cell assembly.
It is also preferred to comprise a step of hardening the support carrier after disposing. This enables the cell to further aggregate to a state of higher density. For example, in the case of collagen gel, it can be solidified by letting it stand under culture temperature for several minutes to several tens of minutes. In this case, less components other than the cell there is in the cell assembly, a state of higher density can be realized.

The "step of culturing the first and second cell assemblies inside a support carrier" in the present invention is for example described in Patent Documents 1 to 4 and Japanese Published Unexamined Patent Application Publication No. 2008-29757, the disclosures of each of these references incorporated herein by reference in its entirety.

The culture period varies depending on for example the number of cells disposed inside the support carrier, the state of the cell assembly, culture conditions, and animal species, as can be arbitrarily selected by those skilled in the art. It is preferred to culture for at least 1 day, more preferably for 3 days or more to allow eruption as a functional tooth when transplanted in the mouth.
By prolonging the culture period, the formation of reconstituted tooth germ such as the accumulation of dentin and enamel, the formation of tooth crown, and the formation of tooth root can be further progressed. In order to obtain the desired state, the culturing may be e.g. for 6 days or more, 30 days or more, 50 days or more, 100 days or more, or 300 days or more, and the medium or culture conditions can also be altered during culture.

The step of culturing inside the support carrier may be culturing the support carrier that encases the first and second cell assemblies alone, or may be culturing in the presence of other animal cells etc.
When the support carrier is cultured alone, the culture conditions can be those employed in general animal cell culturing. A mammal-derived serum may be added to the culture, or various cell factors known to be effective for proliferation or differentiation of these cells may also be added. Examples of these cell factors can include FGF and BMP.

With respect to gas exchange or nutritional supply of the cell assembly, and with respect to no contact or contamination with other animal cells and enabling all steps to be performed in vitro, it is preferred to make culturing inside the support carrier an organ-culturing. In organ-culturing, in general, a porous membrane is floated on a medium suited for animal cell proliferation, and a support carrier that encases the first and second cell assemblies is mounted on the membrane and cultured. The porous membrane employed here is preferably that having numerous pores of about 0.3 to 5 µm, examples of which can include Cell Culture Insert (product name) or Isopore filter (product name).

In the method for manufacturing a regenerated tooth unit employed in the present invention, after producing the regenerated tooth germ, the said regenerated tooth germ can be further cultured in vivo in the body of a mammal. By culturing in vivo in the body of a mammal, the differentiation-induction of the regenerated tooth and formation of the periodontal tissue can be promoted, and a regenerated tooth unit having a periodontal tissue which is more suitable for the present invention can be formed.

The tooth obtained by the method according to the present invention has a cell disposition (structure) specific to tooth which is dentin on the inside and enamel on the outside, preferably also comprises a direction where the tip of the tooth (tooth crown) and the tooth root are in correct positions, and sufficiently serves the functions as a tooth. Accordingly, it can be widely utilized as an alternative to tooth. It can also be employed as a research tool useful in the research to elucidate the developmental process of tooth.

Further, by prolonging the culture period, the periodontal tissue such as the alveolar bone or the periodontal ligament that supports and fixes the tooth on the jaw bone can also be formed in addition to teeth per se. This can further enhance the practicality of the tooth after transplantation. It is also possible to separate and utilize only the periodontal tissue.

The regenerated tooth unit employed in the present invention can be produced by arbitrarily adjusting its size depending on the size of the missing part or the size of tooth to be regenerated. For example, when disposing said mesenchymal cell assembly and said epithelial cell assembly in contact in the production of a regenerated tooth germ, each cell assembly can be formed in a rough cylindrical shape, and in such a case, a tooth having the desired length in one direction can be formed by controlling the contact length of the axial direction of the cylinder. For example, a regenerated tooth having the said desired length can be produced by making the contact length of said mesenchymal cell assembly and said epithelial cell assembly to be ± 25 % of the width of the tooth crown of the desired regenerated tooth.

The tooth configuring the regenerated tooth unit employed in the present invention has a cell disposition (structure) specific to tooth which is dentin on the inside and enamel on the outside, preferably also comprises a direction where the tip of the tooth (tooth crown) and the tooth root are in correct positions, and sufficiently serves the functions as a tooth. Accordingly, it can be widely utilized as an alternative to innate tooth.

Further, the regenerated tooth unit employed in the present invention has a periodontal tissue such as the alveolar bone or the periodontal ligament that supports and fixes the tooth on the jaw bone in addition to teeth per se, and can thereby further enhance the restoration of the jaw bone after transplantation.

The regenerated tooth unit employed the method for restoring the jaw bone according to another embodiment of the present invention can be cultured with a spacer when culturing a regenerated tooth germ in vivo in the body of a mammal. More specifically, after producing a regenerated tooth germ, the regenerated tooth germ can be disposed inside a spacer, and transplanted inside the body of a mammal along with the spacer comprising the regenerated tooth germ and cultured.

A spacer herein means an appliance disposed such that it covers the regenerated tooth germ, so that the regenerated tooth germ does not extend to more than necessary when culturing the regenerated tooth germ in vivo in the body of a mammal. The spacer can also play a role in keeping the regenerated tooth germ from being applied capsular pressure or excessive pressure from a tissue inside the body, for example when culturing inside the body such as the subrenal capsule. The shape of the spacer may be any shape as long as these purposes are achieved.

In other words, the shape of the spacer may be any that can secure the interior space which will allow the development of a regenerated tooth unit having a desired regenerated tooth unit size (mainly length and thickness). The length of the regenerated tooth unit refers to the length from the future occlusal surface site to the future root apex site in the tooth portion of the regenerated tooth unit. It will mean the distance to its tip (the site farthest from the future occlusal surface site) when the periodontal tissue is formed around the future root apex site. The approximate maximum tolerance of the length of the regenerated tooth unit means the extent of the maximum value tolerated as the length of the regenerated tooth unit obtained after culturing the regenerated tooth germ in vivo in the body of a mammal. The said approximate maximum tolerance can be arbitrarily determined by those skilled in the art according to the subject or site to which the regenerated tooth germ is later transplanted. The thickness of the regenerated tooth unit generally refers to the thickness of the tooth crown portion of the regenerated tooth unit, but it may refer to the thickness of the portion having the periodontal tissue when the periodontal tissue is formed around the future root apex site. As with the teeth of human beings, the cross-section of tooth crown portion is ordinarily neither perfect square nor rectangle, but is rather defined by the major and minor axes.

From conventional knowledge, since the direction of extension of the regenerated tooth germ is controllable to a considerable extent, the direction of the regenerated tooth germ composition per se when disposing the regenerated tooth germ composition inside the spacer or its relative position inside the spacer can be arbitrarily adjusted aiming at the desirable extension direction. Accordingly, the extension direction of the regenerated tooth germ composition can be considered in designing the three-dimensional size of the spacer in order to obtain the desired size.
In general, the interior space of the spacer must be larger than the desired regenerated tooth unit. That is to say, the interior space of the spacer has a role in preventing the regenerated tooth unit from extending to more than the maximum tolerance.

A case where the shape of the spacer is a circular cylinder (ring-shaped) with open top and bottom will be described below. Those skilled in the art, however, may easily design a spacer having the desired shape based on the disclosures herein etc.
In case of such a circular cylinder-shape (ring-shape) with open top and bottom, a nutritional supply route from the exterior of the spacer is also sufficiently secured through the opening at the top and bottom, and the formation of a regenerated tooth unit is enabled.
In addition, when employing such a circular cylinder-shaped (ring-shaped) spacer, setting the extension direction of the regenerated tooth germ as the lateral direction of the circular cylinder ring (i.e. the radial direction of the circular cylinder), the longitudinal direction of the circular cylinder (i.e. the height of the circular cylinder) may be set so that the desired thickness of the occlusal surface of the regenerated tooth unit is obtained and the maximum tolerance is not exceeded. Also in this case, the longitudinal direction of the circular cylinder (i.e. the height of the circular cylinder) may be set so that the periodontal tissue thickness of the desired regenerated tooth unit is obtained and the maximum tolerance is not exceeded. The desired thickness or the maximum tolerance in this case can be determined based on the size or shape of the missing tooth part of the target animal. It is preferred that the shape of the periodontal tissue of the regenerated tooth unit fits the size or shape of the missing part that will be the subject of transplantation since it will allow easier success of transplantation and integration of the bone. In addition, the inner diameter of the ring may be set aiming at the approximate maximum tolerance of the length of the regenerated tooth unit in order to prevent the regenerated tooth unit from extending to more than necessary.
In case of employing a spacer with such a circular cylinder-shape, if the radial direction of the ring is set as the extension direction, when the regenerated tooth germ is disposed so that the longitudinal direction (height direction) will be the width of the tooth crown of the regenerated tooth unit, the height of the cylinder is for example 3-15 mm, preferably 5-12 mm when culturing a human tooth, and 0.4-2.0 mm, preferably 0.6-1.8 mm when culturing a mouse tooth, and its inner diameter (inside diameter) is 10-30 mm, preferably 15-26 mm when culturing a human tooth, and 0.9-2.0 mm, preferably 1.2-1.9 mm when culturing a mouse tooth.

Further, when utilizing a rough circular cylinder-shaped spacer, by disposing the future occlusal surface site of the regenerated tooth germ in great vicinity of one inner wall of the spacer facing the inner wall, and disposing the future root apex site towards the radial direction of the cylinder, the regenerated tooth germ is not applied excessive pressure from a tissue inside the body, the regenerated tooth germ is prevented from extending to more than necessary, and is communicatable with the exterior and substance supply route can be secured.
The occlusal surface of the regenerated tooth germ can grow in a natural shape without being crushed by pressure, the tooth will extend in the diameter direction of the cylinder, and in general, the extension can be stopped at a length shorter than the maximum tolerance before or after reaching the opposite wall.
The regenerated tooth germ can avoid the pressure in the horizontal direction of the cylinder by virtue of the spacer wall, as well as mostly avoid the pressure in the top-bottom direction by virtue of the top and bottom edges of the spacer.

Those skilled in the art can employ a spacer of any desired shape without being limited to a circular cylinder-shape in the manufacturing of a regenerated tooth unit in vivo, in order to achieve the desired purpose defined herein, or in order to achieve other proposes necessary in some instances. Examples include, but are not limited to, those having an elliptic or polygonal base instead of a circular cylinder, or those having a sphere shape.

The spacer that can be employed in the present invention is configured such that the regenerated tooth germ disposed inside is communicatable with the exterior of the spacer. This is because since the regenerated tooth germ receives a supply of nutrients etc. from a tissue inside the body of a mammal or receives actions of cytokines produced by the animal cell, it is necessary to secure a supply route for these substances to reach the regenerated tooth germ inside the spacer. In order for it to be communicatable, the shape of the spacer, regardless of whether large or small or the number, can form pore(s) sufficient for supplying the substance. For example, it may have a partial large pore such as in the case of a cylinder, or it may have numerous small pores such as in the case of a mesh throughout the spacer. In addition, it may be without a pore as long as the substance can penetrate the material of the spacer.

When manufacturing the regenerated tooth unit employed in the present invention with a spacer, the mammal employed for culturing the regenerated tooth germ is not particularly limited as long as it is able to culture a regenerated tooth germ in vivo inside the body, and may be a non-human mammal. Examples include a cow, a horse, a pig, a dog, a cat, and a mouse, preferably a pig and a mouse. It is also particularly preferred that it is the same species as the tooth germ tissue. When transplanting and culturing in an animal that is not the same species as the tooth germ tissue, it is preferred to employ an animal modified to be immunodeficient. In order to allow the development of an organ or tissue of an animal cell to be as normal as possible, the subrenal capsule, the mesenterium, and the subcutaneous etc. are preferred as the favorable in vivo body site for in vivo growth.
The culture period after transplantation varies depending on the size of tooth at the time of transplantation and the size of tooth to be developed, and can generally be between 3 to 400 days. For example, when transplanting in the subrenal capsule, although varied depending on the size of the tooth germ to be transplanted and the size of tooth to be regenerated, about 7 to 60 days is preferred.

The material of the spacer is not particularly limited and it may be any having sufficient rigidity against the pressure from a tissue inside the body, examples of which include gold, silver, iron, copper, aluminum, polyethylene, resin, dental resin, and dental cement.

In addition, in the method for manufacturing a regenerated tooth unit employed in the present invention, the step of disposing and culturing a regenerated tooth germ inside a spacer is preferably performed by filling the spacer with a support carrier, and disposing the regenerated tooth germ inside the said support carrier.
The support carrier is not particularly limited as long as it enables cell culture inside, but is preferably e.g. gel, fiber, or solid, and excessive pressure can be further prevented from being applied to the regenerated tooth germ in vivo by employing such support carrier.
Examples of a support carrier employed for manufacturing the regenerated tooth unit employed in the present invention include collagen, agarose gel, carboxymethylcellulose, gelatin, agar, hydrogel, Cellmatrix (product name), Mebiol Gel (product name), Matrigel (product name), elastin, fibrin, laminin, extracellular matrix mixture, polyglycolic acid (PGA), polylactic acid (PLA), and lactic acid/glycolic acid copolymer (PLGA). Among these, collagen, agarose gel, carboxymethylcellulose, gelatin, agar, hydrogel, Cellmatrix, Mebiol Gel, Matrigel, extracellular matrix mixture, elastin, fibrin, and laminin having appropriate hardness or retentivity are preferred.
For example, a liquid support carrier may be employed and hardened after disposing the regenerated tooth germ. For example, after filling the spacer with a collagen solution and disposing the regenerated tooth germ into the collagen solution, the collagen can be gelled by culturing at 37°C in a CO₂ incubator. The regenerated tooth germ can then be disposed in vivo in the body of a mammal along with the spacer and cultured.

In another embodiment of the present invention, according to the method of manufacturing the regenerated tooth unit employed in the present invention with a spacer, when culturing a regenerated tooth germ in vivo, a tooth having a shape compatible to the missing part is easily manufactured because the tooth can be grown to a natural shape without being applied pressure in vivo and crushed. In addition, for a regenerated tooth unit manufactured with a spacer, not only the tooth portion but also the periodontal tissue portion will not be crushed in vivo by being applied pressure, and thus the periodontal tissue can be sufficiently developed, and a regenerated tooth unit with more alveolar bone formation can be manufactured. Accordingly, a regenerated tooth unit manufactured with a spacer can be employed as that more suitable for restoring the alveolar bone.

In addition, in another aspect of the present invention, the regenerated tooth unit employed in the present invention is characterized in that it is manufactured by a method comprising a step of culturing regenerated tooth germ in vivo in the body of a mammal, wherein mechanical stimulation is applied from the exterior of the mammal when culturing the regenerated tooth germ.
The formation of the periodontal ligament in the regenerated tooth unit is promoted by applying mechanical stimulation. Examples of mechanical stimulation include, but are not limited to, vibration, compression, and friction. For example, it is preferred to apply vibration at a near sonic wave frequency (e.g. about 20000 Hz to 40000 Hz). Accordingly, it is believed that better restoration of the alveolar bone is promoted by transplanting a regenerated tooth unit in which the formation of the periodontal ligament has been promoted by applying mechanical stimulation.

Moreover, in the method of manufacturing the regenerated tooth unit employed in the present invention with the above stated spacer, it is also preferred that mechanical stimulation is applied from the exterior of the mammal when culturing the regenerated tooth germ in vivo.

In the method for restoring the alveolar bone according to the present invention, depending on the tooth regeneration stage of the regenerated tooth unit to be transplanted, it is preferred to dispose so that the tooth crown is on the interior side of the mouth if the formation of tooth crown is already recognized. If it is at a stage where the formation of tooth crown is not recognized, it is preferred to dispose so that the epithelial cell layer corresponding to the tooth crown portion or the epithelial cell layer of the regenerated tooth germ is on the interior side of the mouth. This enables the tip part of the tooth (tooth crown) to be on the interior side of the mouth to have a direction similar to the surrounding teeth, and integration of the periodontal tissue portion of the regenerated tooth unit and the jaw bone can be attempted.

The regenerated tooth unit employed in the present invention can also be employed in a method for repairing a missing tooth of an animal. The method for repairing a missing tooth of an animal comprises a step of transplanting said regenerated tooth unit to the missing tooth part. It is possible to manufacture and transplant a regenerated tooth unit compatible to the size and shape of the missing part.
According to a method for manufacturing a regenerated tooth unit with a spacer, when culturing a regenerated tooth germ in vivo, a tooth having a shape compatible to the missing part is easily manufactured because the tooth can be grown to a natural shape without being applied pressure in vivo and crushed.
In addition, it is desired to produce a regenerated tooth having the same length as the missing tooth for transplanting to the missing part, and according to the method for manufacturing a tooth according to the present invention, the maximum value of the length of the tooth can be controlled. When the length of the tooth exceeds the maximum tolerance, the tooth cannot be transplanted as it is, but if it is shorter than the maximum tolerance, it is possible to allow it to grow after transplantation to obtain a regenerated tooth of a desired length.

The terms used herein are for the purpose of describing particular embodiments and do not intend to limit the invention.
In addition, the term "comprising" as used herein, unless the context clearly indicates to be understood otherwise, intends the presence of the described items (such as components, steps, elements, and numbers), and does not exclude the presence of other items (such as components, steps, elements, and numbers).
Unless otherwise defined, all terms used herein (including technical and scientific terms) have the same meaning as that broadly recognized by those skilled in the art of the technology to which the present invention belongs. The terms used herein, unless explicitly defined otherwise, should be construed as having meanings consistent with the meanings herein and in related technical fields, and are not to be construed as idealized or excessively formal meanings.
The embodiments of the present invention may be described referring to schematic diagrams. In such a case, they may be exaggerated in presentation in order to allow clear description.
Terms such as first and second are employed to express various elements, but it should be recognized that these elements are not to be limited by these terms. These terms are employed solely for the purpose of discriminating one element from another, and for example, it is possible to describe a first element as a second element, and similarly, to describe a second element as a first element without departing from the scope of the present invention.

### Examples

The present invention will now be described in further detail referring to Examples. However, the present invention can be embodied by various aspects, and shall not be construed as being limited to the Examples described herein.

### <Example 1: Production of Regenerated Tooth Unit with Spacer>

As an example of the method for producing the regenerated tooth unit employed in the present invention, after producing a regenerated tooth germ, in vivo culturing was performed in vivo in the body of a mammal, and a regenerated tooth unit was produced. In vivo culturing of regenerated tooth germ with a spacer was performed with the purposes of avoiding the influence of renal capsular pressure in subrenal capsule transplantation and producing a regenerated tooth unit which is suitable for the shape of the missing tooth part and in which the periodontal tissue is sufficiently developed.

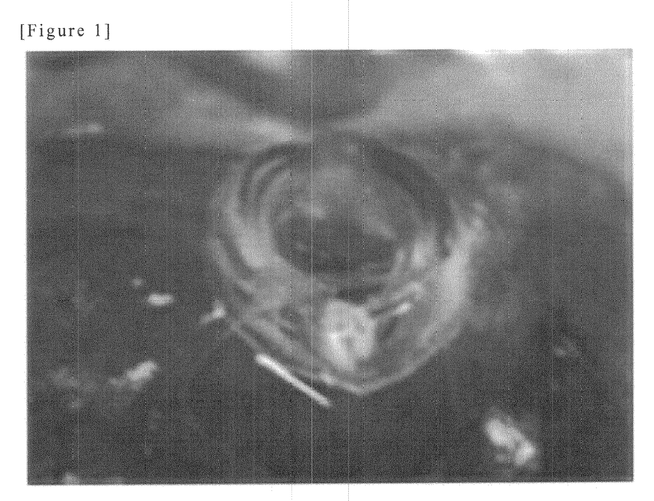

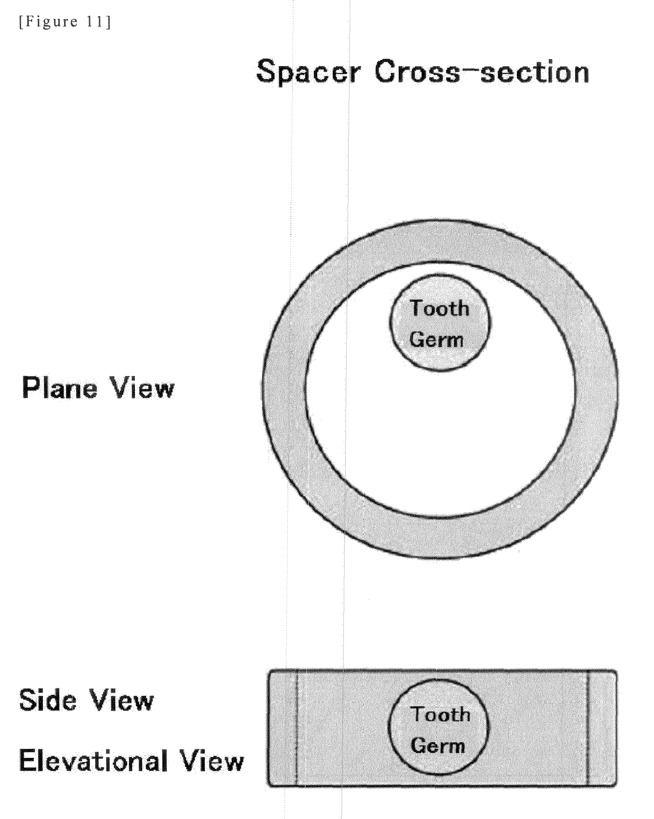

A micropipette chip (HRI-110 NEW, Molecular Bio Products, San Diego, CA, USA) was cut in a ring-shape (cylinder-shape) so that the inner diameter (inside diameter) was 1.3 mm and the height was 1.3 mm to fabricate a spacer, and filled the interior with a collagen solution and used. The regenerated tooth germ was disposed so that the spacer wall surface was in proximity to the tooth crown side in order to promote extension in the tooth root direction, and cultured in a CO₂ incubator (SANYO Electric Co., Ltd, Osaka, Japan) at 37°C to harden the collagen gel. Next, the spacer comprising a regenerated tooth germ was transplanted to the bilateral subrenal capsule of a 7 weeks-old C57BL/6 mouse. Transplantation to the subrenal capsule was performed by making a 2 to 3 mm incision in the renal capsule, detaching the renal capsule and the renal parenchymal, and inserting between them the spacer comprising a regenerated tooth germ. As a comparative example, a regenerated tooth germ organ-cultured for 7 days was transplanted to the subrenal capsule without a spacer. The photograph at the time of transplantation is shown in Figure 1. The schematic diagram of the positional relationship between the spacer and the regenerated tooth germ at the time of transplantation is also shown in Figure 11.

Micro CT images were taken 3 weeks after transplantation, and the image data were analyzed with an integrated image processing software. The maximum diameter in the tooth crown portion was defined as the major axis and the maximum diameter orthogonal to the major axis was defined as the minor axis, and each length was measured and the ratio thereof calculated.

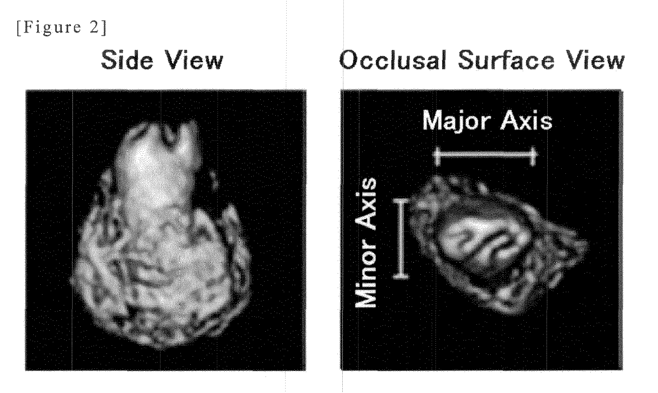

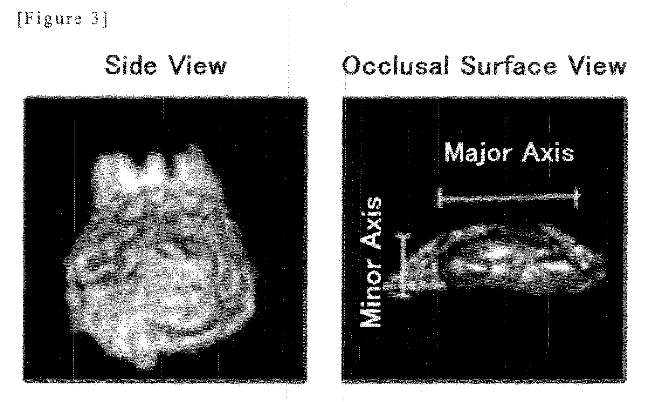

An example of culturing with a spacer is shown in Figure 2, and an example of culturing without is shown in Figure 3. While the regenerated tooth unit cultured with a spacer was in a state close to the normal tooth, the regenerated tooth unit cultured without a spacer had a major axis extremely longer compared to the minor axis, having a flattened shape.

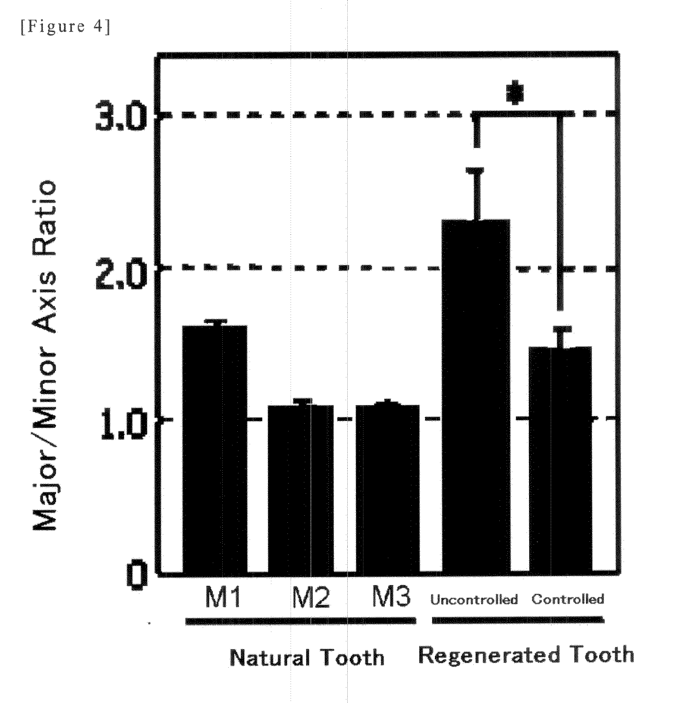

The major and minor axes were measured for each of the 7 cases of the group cultured with a spacer (controlled group) and the group cultured without a spacer (uncontrolled group). The result of calculating the major/minor axis ratio is shown in Figure 4 (right). The major/minor axis ratios of the regenerated tooth unit were 1.46 ± 0.16 mm for the group cultured with a spacer (controlled group: Examples), and 2.30 ± 0.35 mm for the group cultured without a spacer (uncontrolled group: Comparative Examples).

Figure 4 (left) also shows the result of measuring the maximum diameter in the tooth crown portion of the first, second, and third molar of the lower jaw (M1, M2, and M3) of adult mouse defined as the major axis and the maximum diameter orthogonal to the major axis defined as the minor axis, and calculating the major/minor axis ratio. The major/minor axis ratios of M1, M2, and M3 were 1.61 ± 0.05 (n = 5), 1.09 ± 0.04 (n = 5), and 1.12 ± 0.04 (n = 5), respectively.

Consequently, it was confirmed that the shape of the regenerated tooth unit cultured in vivo without a spacer becomes flattened whereas the shape of the regenerated tooth unit cultured in vivo with a spacer will not be flattened and a significant difference exists between the major/minor axis ratios of the two, and that the major/minor axis ratio of the regenerated tooth unit cultured in vivo with a spacer is equivalent to natural teeth (Student's t-test, *p<0.0001). In addition, it was confirmed that the shape will not be flattened when cultured in vivo with a spacer as compared to when not only the tooth crown portion of the regenerated tooth unit but also the periodontal tissue portion of the regenerated tooth unit was cultured in vivo without a spacer, and a regenerated tooth unit having a shape where the periodontal tissue is spread in both directions of the minor and major axes directions of the tooth crown in the figure is obtained (see the figures on the right side of Figures 2 and 3). Accordingly, the shape of the periodontal tissue can also be controlled by altering the shape of the spacer (in this Example, the height of the circular cylinder of the spacer). By adjusting the size or shape of the spacer, producing and transplanting a regenerated tooth unit having a periodontal tissue that fits the shape of the missing part will enable the success of transplantation, and in turn be useful in restoring the alveolar bone of the transplanted animal. It is also believed that by employing a spacer, a more rigid support of the regenerated tooth by the alveolar bone will be possible in a regenerated tooth unit where the periodontal tissue has the shape that is spread in both directions of major and minor axes of tooth crown.

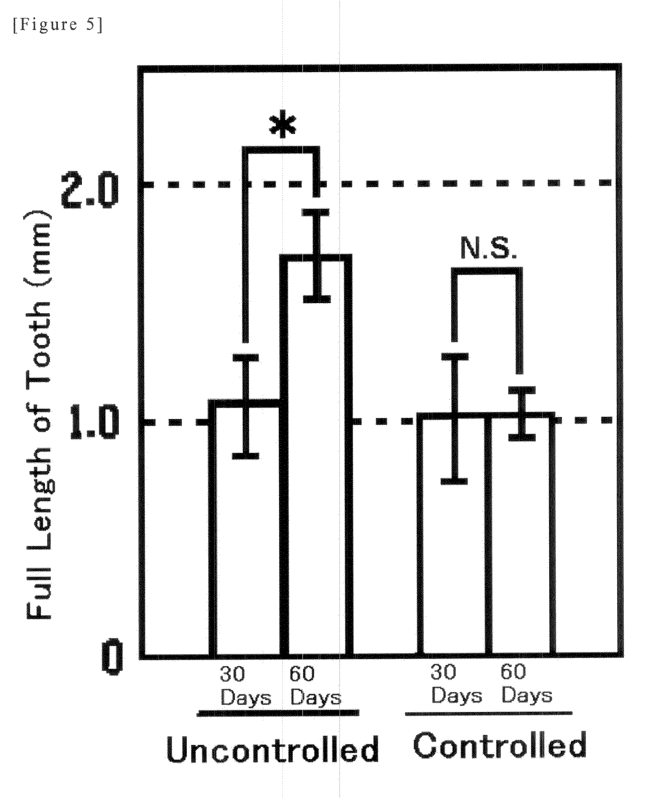

The regenerated tooth unit on Day 30 and Day 60 of subrenal capsule transplantation was also harvested, and micro CT images were taken and analyzed by an integrated image processing software to measure the length. The length from the cusp to the root apex of the tooth was measured on the micro CT image without including the alveolar bone region formed.
The result is shown in Figure 5. In the uncontrolled group without a spacer, the cusp-root apex length was 1.07 ± 0.20 mm (n = 6) on Day 30 as opposed to 1.70 ± 0.26 mm (n = 6) on Day 60, and the length of the tooth had significantly increased. On the other hand, in the controlled group with a spacer, as seen from 1.01 ± 0.19 mm (n = 13) on Day 30 and 1.02 ± 0.11 mm (n = 10) on Day 60, increase in the length of the tooth due to the number of transplantation days was not observed (Student's t-test, *p<0.0001).
Consequently, it became clear that the length of the regenerated tooth can be arbitrarily adjusted by culturing a regenerated tooth germ in vivo with a spacer.

### <Example 2: In Vivo Culture of Regenerated Tooth Germ with Mechanical Stimulation>

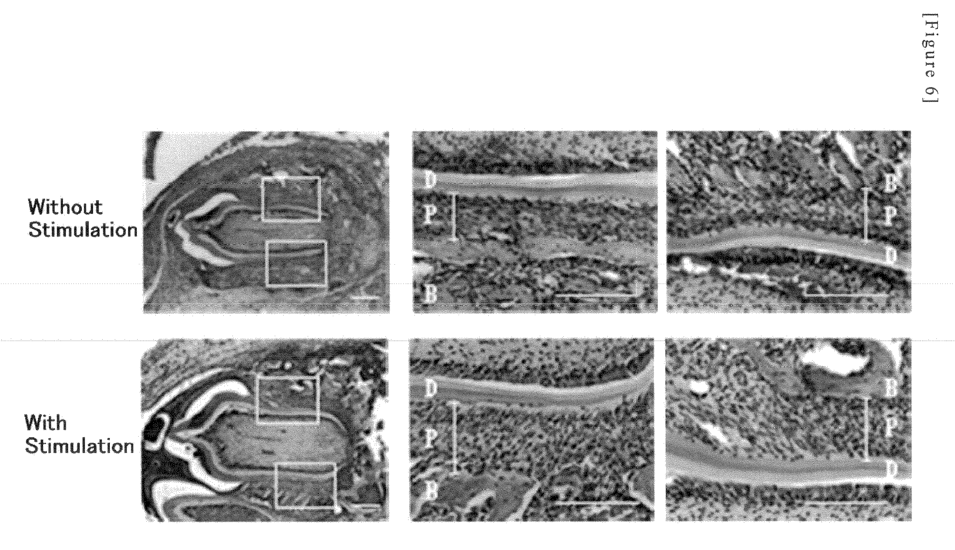

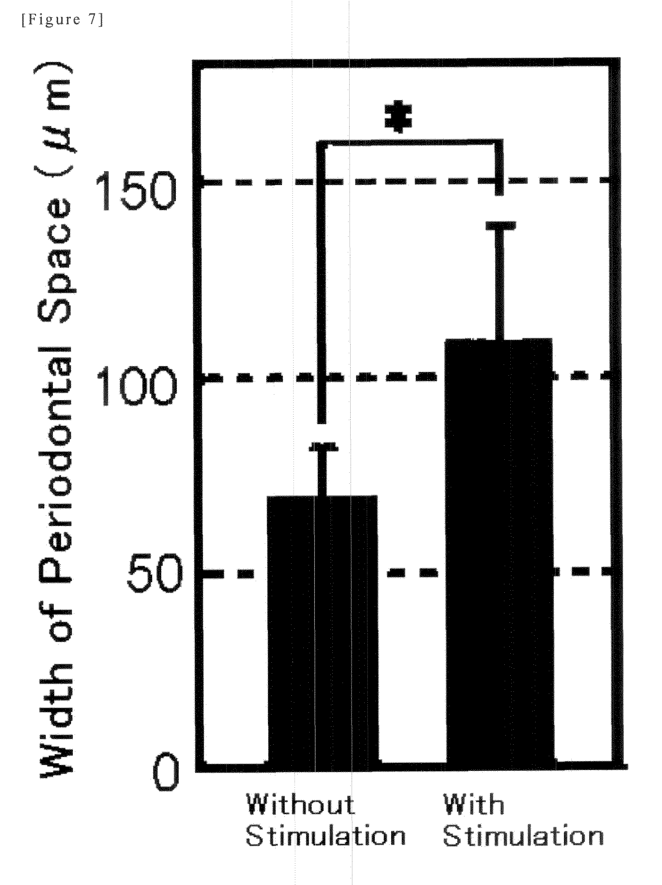

In accordance to the method of Example 1, a regenerated tooth germ organ-cultured with a spacer was transplanted in the subrenal capsule along with the spacer. As mechanical stimulation, a sonic wave electric toothbrush (frequency 31,000 Hz: Doltz™, Panasonic) was pressed onto the mouse dorsal skin at a pressure of 3.9 x 10⁴ Pa (5 minutes/day) from Day 7 after subrenal capsule transplantation. No mechanical stimulation was given to the control group, and these were harvested after 30 days of transplantation period as comparison subjects.
Micro CT images were taken for the regenerated tooth unit harvested with a method similar to Example 1, and the width of the periodontal space (sometimes simply referred to as "the width of the periodontal ligament") was measured using a three-dimensional image analysis software. Specifically, the distance between the tooth root surface of the regenerated tooth unit and the alveolar bone formed on the three-dimensional image was set as the width of the periodontal space, and six points in each of the cross-sections on the major/minor axis were measured. The regenerated tooth was then fixed in 4 % paraformaldehyde (Paraformaldehyde: PFA), decalcified in 10 % ethylenediaminetetraacetic acid (EDTA), and embedded in paraffin. Continuous sections 8 micrometers thick were then created, hematoxylin-eosin (HE) stained, and histological evaluation was performed.
The result of histological evaluation is shown in Figure 6. In the figures, B shows the alveolar bone, D shows the dentin, and P shows the periodontal ligament, and the bar next to the letter P in the figure shows the width of the periodontal ligament. The scale bar in the lower right of the figures shows 100 µm. The periodontal ligament of the group with stimulation was formed thicker than the group without stimulation, and ordered sequence of cells in the periodontal ligament region was recognized.
The result of measuring the width of the periodontal ligament on the CT image is also shown in Figure 7. The width of the periodontal ligament of the group with stimulation was 108.5 ± 30.6 µm (n = 33) and the width of the periodontal ligament of the group without stimulation was 68.7 ± 14.1 µm (n = 12) (Student's t-test, *p<0.0001).
From the above result, it was found that a regenerated tooth unit having sufficient periodontal ligament tissue is obtained by applying mechanical stimulation from the exterior when culturing a regenerated tooth germ in vivo.

### <Example 3: Jaw Bone Transplantation of Regenerated Tooth Unit Obtained by In Vivo Culture>

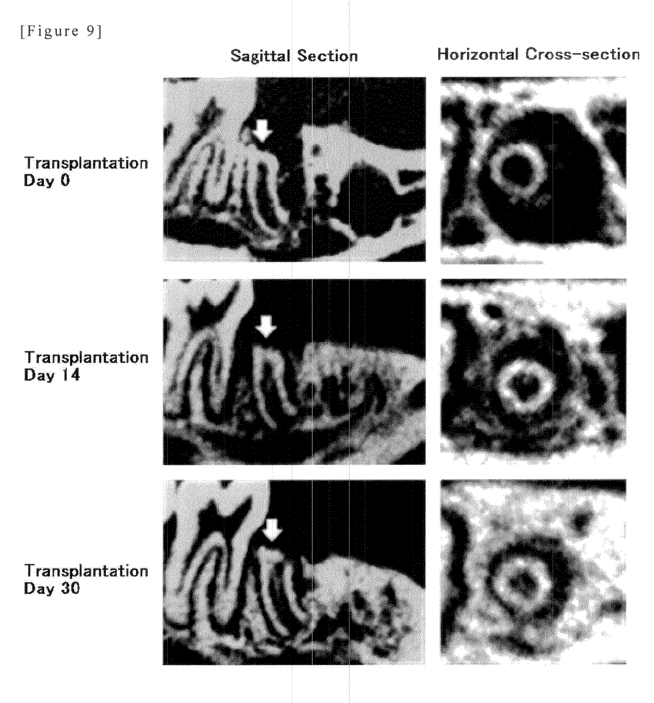

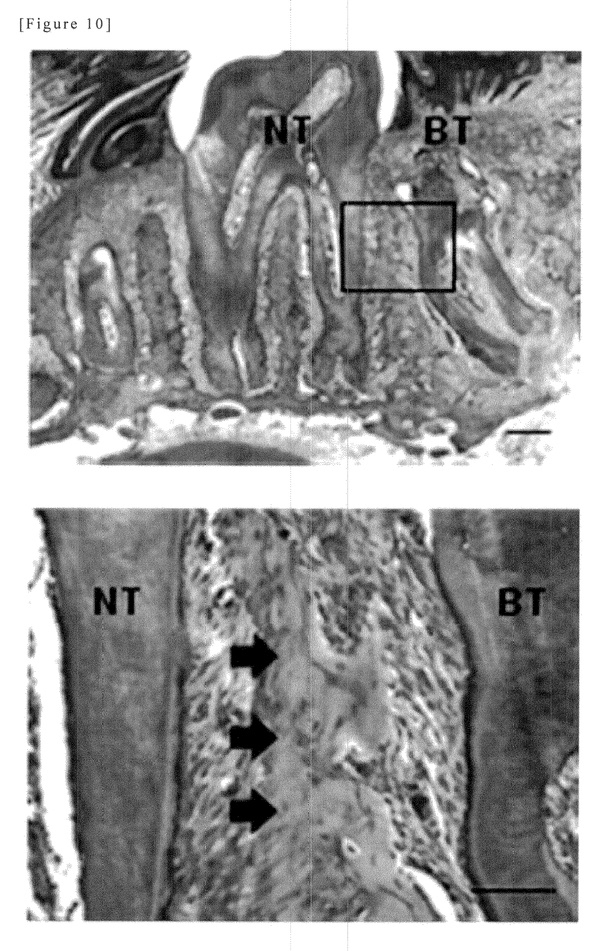

The first molar of the lower jaw of a 4 weeks-old C57BL/6 mouse was extracted, and 3 days of healing period was provided. Gingival incision/detachment of the same portion was then performed, the alveolar bone was cut with a dental micromotor (Viva-Mate Plus, Nakanishi Inc., Tokyo, Japan), and a transplantation fossa having a mesiodistal diameter of 1 mm and a buccolingual diameter of 0.8 mm was formed.
A regenerated tooth unit produced by culturing with a spacer for 30 days in the subrenal capsule according to Example 1 and applying mechanical stimulation during culture according to Example 2 was embedded in the jaw bone transplantation fossa, and the gingiva was sutured with an 8-0 nylon surgical suture (Bear Medic Corporation, Chiba, Japan).

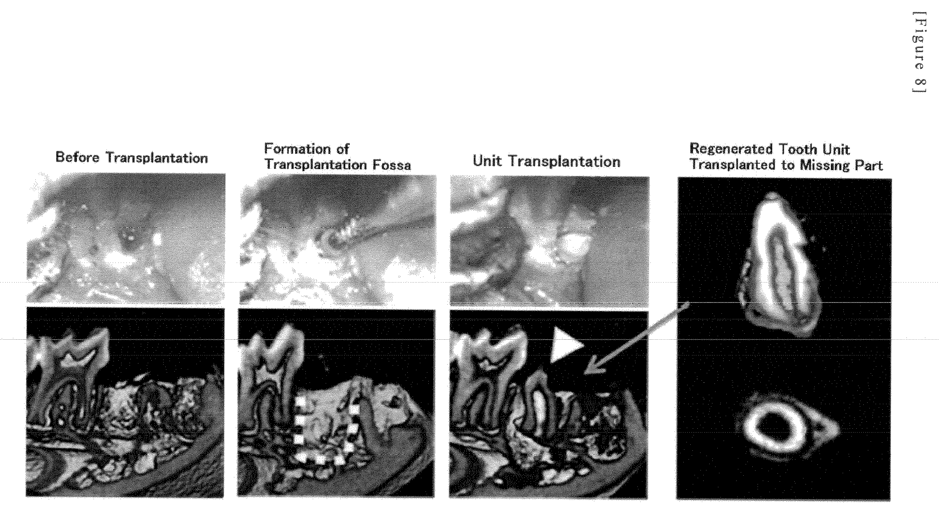

Figure 8 shows the appearance of the formation of the transplantation fossa and the transplantation of a regenerated tooth unit.
Micro CT images of a mouse with transplanted regenerated tooth unit were taken on transplantation Day 0, Day 14, and Day 30 with a method similar to above, and the attachment between the graft and the recipient alveolar bone was evaluated. The jaw bone comprising the graft was then fixed with 4 % PFA, decalcified with 10 % formic acid-sodium citrate decalcification solution, and embedded in paraffin, after which continuous sections 8 µm thick were created and histological evaluation was performed by HE staining.
The CT image is shown in Figure 9, and the HE staining image is shown in Figure 10.
As shown in Figure 9, partial attachment was observed on transplantation Day 14 between the graft and the recipient's second molar alveolar bone of the lower jaw, and bone attachment was observed in almost the entire circumference on transplantation Day 30. In the figure, the arrow shows the regenerated tooth unit.
As shown in Figure 10, the possibility that the alveolar bone of the interalveolar septum between the regenerated tooth and the second molar of the lower jaw are fused and that the regenerated tooth unit is integrated with the alveolar bone of the transplantation site via bone attachment was shown in the tissue image on transplantation Day 30. Also from tissue analysis, although calcification was observed on a part of the regenerated tooth dental pulp as an accidental symptom of transplantation (3 out of 16 cases), the periodontal ligament of the regenerated tooth unit was maintained after transplantation, and no bone adhesion was observed (0 out of 16 cases). In the figure, the arrow shows integration by bone attachment, the scale bar shows 100 µm, BT shows the regenerated tooth, and NT shows the natural second molar of the lower jaw.
Consequently, it was shown that a regenerated tooth unit manufactured with the manufacturing method of the present invention is integrated well when transplanted to the jaw bone.

### <Example 4: Transplantation of Regenerated Tooth Unit to Diffuse Bone Missing Model>

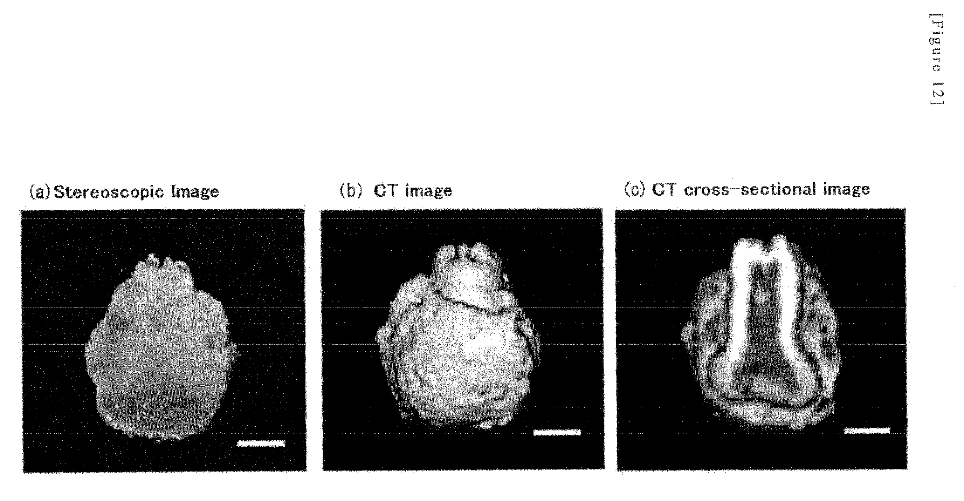

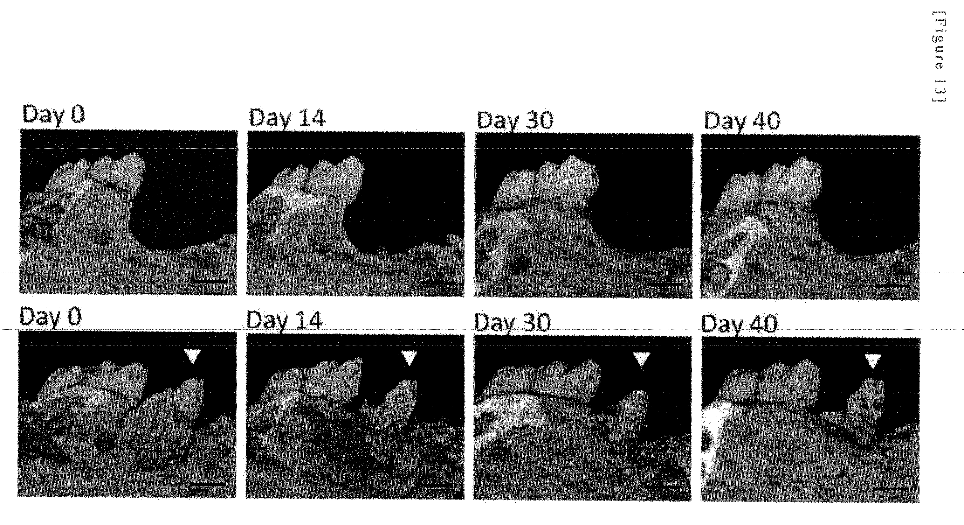

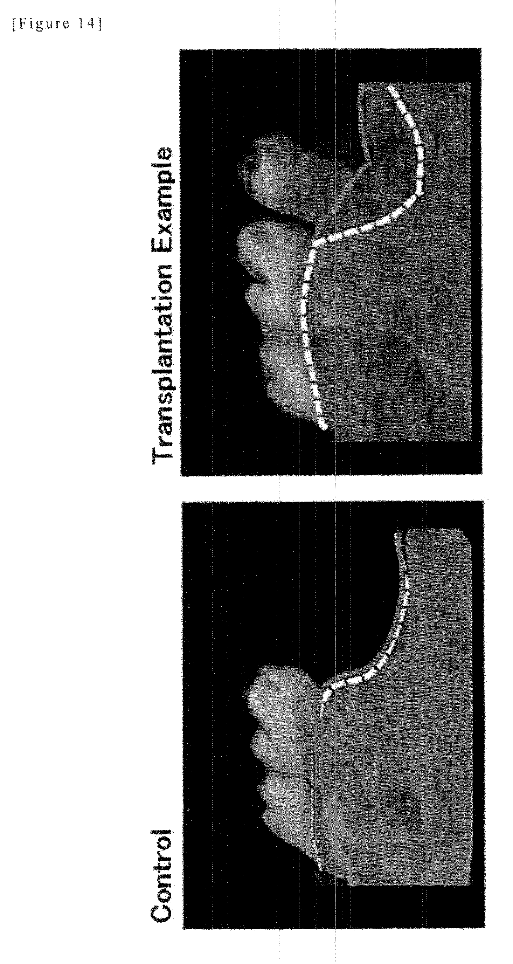

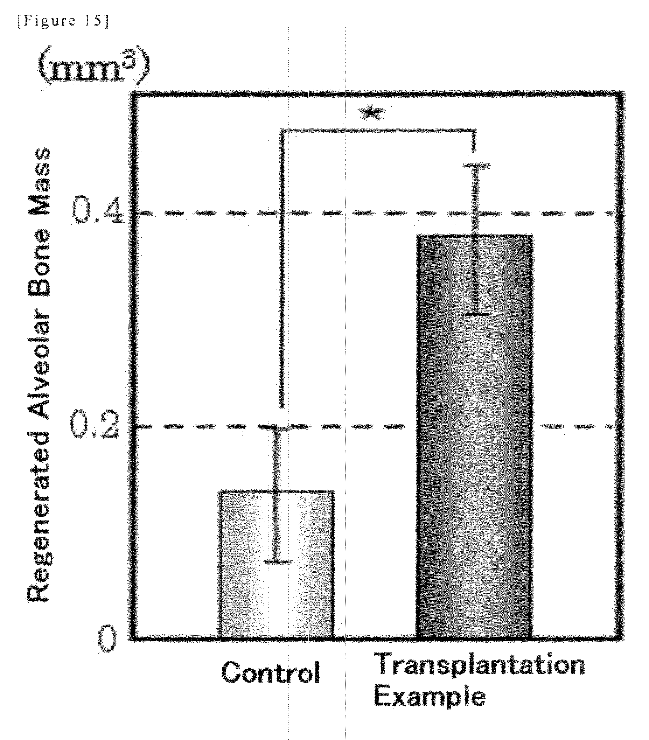

The first molar of the lower jaw of a 4 weeks-old C57BL/6 mouse was extracted, and 3 days of healing period was provided. Gingival incision/detachment of the same portion was then performed, the alveolar bone was cut with a dental micromotor (Viva-Mate Plus, Nakanishi Inc., Tokyo, Japan), and three-walled diffuse bone missing model having a mesiodistal diameter of 2.5 to 3 mm, a buccolingual diameter of 2 mm, and a depth of 3 mm was created. A regenerated tooth unit produced by disposing the regenerated tooth germ inside the spacer and subjecting to subrenal capsule transplantation for 50 to 60 days by a method similar to Example 1 was transplanted to the bone missing part, and the gingiva was sutured with an 8-0 nylon surgical suture (Bear Medic Corporation, Chiba, Japan). Figure 12 shows the stereoscopic image, CT appearance image, and CT cross-sectional image of the transplanted regenerated tooth unit.
Micro CT images of a mouse with transplanted regenerated tooth unit were taken on transplantation Day 0, Day 14, Day 30, and Day 40, and the attachment between the graft and the alveolar bone of the recipient, as well as the vertical bone regeneration of the buccal alveolar bone were evaluated. Micro CT images of the regenerated tooth unit and the jaw bone with the regenerated tooth unit transplanted were taken with a 3D micro X-ray CT R_mCT for experimental animals (Rigaku Corporation) under conditions of 90 kV, 150 mA, and section thickness of 10 mm, and image construction and analysis were performed with an image filing software for small animals i-VIEW Type R and high definition 3D/4D image analysis software Imaris (Bitplane). Figure 13 shows the CT images on transplantation Day 0, Day 14, Day 30, and Day 40 of the control (without transplantation of a regenerated tooth unit) and Example (with transplantation of a regenerated tooth unit).
When the regenerated tooth unit immediately after transplantation and 45 days after transplantation were observed with micro CT images, significant restoration of the buccal alveolar bone mass by transplantation of regenerated tooth unit was recognized and integration of the regenerated tooth unit was also shown compared to the control in which the regenerated tooth unit was not transplanted. Figure 14 shows the CT images 45 days after transplantation for the control (without transplantation of a regenerated tooth unit) and Example (with transplantation of a regenerated tooth unit). In the images, the top edge of the alveolar bone immediately after transplantation is shown by a dotted line, and the top edge of the alveolar bone 45 days after transplantation is shown by a solid line.
Further, the regenerated alveolar bone mass at 45 days of transplantation was measured for the control (without transplantation of a regenerated tooth unit) and Example (with transplantation of a regenerated tooth unit). In other words, the regenerated alveolar bone mass was measured by taking the micro CT image of the missing site or the transplantation site over time and analyzing this by an integrated image processing software in order to measure the volume of the buccal alveolar bone which increased from before to after transplantation. The measured result is shown in Figure 15. By measuring the regenerated alveolar bone mass, the regenerated alveolar bone mass was shown to be significantly increased in the example of transplanting a regenerated tooth unit compared to the control without transplantation of the regenerated tooth unit.
Further, in another Example, CT images on transplantation Day 0 and transplantation Day 49 for the control (without transplantation of a regenerated tooth unit) and Example (with transplantation of a regenerated tooth unit) are shown in Figure 16. In the right image in Figure 16, the horizontal line of the top edge of the alveolar bone on 49 days after transplantation of the Example is shown in the photographs of the control and the Example by a dotted line. In the CT photograph of the control, the difference in the vertical direction between the top edge line of the actual alveolar bone and the top edge line of the alveolar bone for the Example is shown by a two-headed arrow. As shown in this figure, it was shown that the alveolar bone is restored in vertical direction by transplantation of a regenerated tooth unit to the missing tooth part.
Accordingly, it was suggested that alveolar bone regeneration of the missing part, and simultaneously tooth regeneration by bone attachment is also possible by transplanting a regenerated tooth unit to the diffuse bone missing part which is ordinarily impossible to transplant an implant or tooth germ.

## Claims

1. A method for restoring the alveolar bone of a mammal with a missing tooth, comprising a step of transplanting a regenerated tooth unit to said missing tooth site.

2. The method for restoring the alveolar bone of a mammal with a missing tooth according to claim 1, **characterized in that** said animal is a non-human mammal.

3. The method for restoring the alveolar bone according to claim 1 or claim 2, **characterized in that** it comprises the steps of:
(a) producing a regenerated tooth germ;
(b) culturing said regenerated tooth germ in vivo in the body of a mammal, and producing a regenerated tooth unit; and
(c) transplanting said regenerated tooth unit to the missing tooth site of the mammal.

4. The method for restoring the alveolar bone according to any one of claims 1 to 3, **characterized in that** it comprises the steps of:
(a) producing a regenerated tooth germ;
(b) culturing said regenerated tooth germ in vivo in the body of a mammal which is an individual that is different from the subject for jaw bone restoration, and producing a regenerated tooth unit; and
(c) transplanting said regenerated tooth unit to the missing tooth site of a mammal that will be the subject for jaw bone restoration.

5. The method for restoring the alveolar bone according to any one of claims 3 to 4, **characterized in that**
said regenerated tooth germ is cultured and maintained by disposing a first cell assembly composed substantially of mesenchymal cells and a second cell assembly composed substantially of epithelial cells in close contact inside a support carrier, wherein at least either one of mesenchymal and epithelial cells is tooth germ-derived.

6. The method for restoring the alveolar bone according to any one of claims 3 to 5, **characterized in that**
said (b) step is culturing the regenerated tooth germ inside a spacer disposed in vivo in the body of a mammal.

7. The method for restoring the alveolar bone according to any of claims 3 to 6, **characterized in that**
mechanical stimulation is applied from the exterior of the mammal in said (b) step.

8. A regenerated tooth unit for restoring the alveolar bone for employing in the method for restoring the alveolar bone according to any one of claims 1 to 7.

9. A regenerated tooth germ for producing the alveolar bone for employing in the method for restoring the alveolar bone according to any one of claims 1 to 7.
